# EUROPEAN PATENT SPECIFICATION

(11) **EP 4 537 864 B1**
(45) Date of publication and mention of the grant of the patent: **25.11.2026**
(21) Application number: 23203655.8
(22) Date of filing: 13.10.2023
(51) Int. Cl.: A61M 1/16, A61M 1/34, A61M 1/36

(54) **EXTRACORPOREAL BLOOD TREATMENT APPARATUS**
EXTRAKORPORALE BLUTBEHANDLUNGSVORRICHTUNG
APPAREIL DE TRAITEMENT EXTRACORPOREL DU SANG

(43) Date of publication of application: 16.04.2025
(73) Proprietor: Gambro Lundia AB, 226 43 Lund (SE)
(72) Inventor: POUCHOULIN, Dominique, 01390 Tramoyes (FR)
(74) Representative: PGA S.p.A., Milano, Succursale di Lugano

(56) References cited:
- US-A1- 2015 122 712
- US-A1- 2022 054 724

## Description

### BACKGROUND

The present disclosure relates to medical fluid treatments and in particular to extracorporeal blood treatments, such as dialysis fluid treatments.

Due to various causes, a person's renal system may fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with kidney failure therapies.

Kidney failure therapies include for instance Hemodialysis (HD), Hemofiltration (HF), Hemodiafiltration (HDF).

The apparatus of the present invention may also be used in the context of continuous renal replacement therapies (CRRT) with or without anticoagulation, for example CRRT with or without systemic anticoagulation (e.g., heparin) / with or without regional anticoagulation (e.g., citrate).

Irrespective of the type of therapy and/or treatment referred to, in-line sensing technology plays a relevant role in the efficient functioning of the extracorporeal blood treatment apparatuses.

It is known from prior art document US2022054724 methods and systems for providing continuous renal replacement therapy (CRRT) to a patient.

The system includes a hemofiltration unit that provides continuous veno-venous hemofiltration (CWH), a replacement fluid flow regulator configured to regulate the flow of a replacement fluid, an anticoagulant flow regulator configured to pump an anticoagulant, and a blood warmer.

Such prior art document provides two significant embodiments. Each embodiment refers to an extracorporeal blood purification (CRRT) system having an extracorporeal blood circuit, which comprises: a hemofilter; a withdrawal access point/line, a return line, an anticoagulant source for supplying anticoagulant substance (heparin, warfarin, rivaroxaban, dabigatran, apixaban, citrate, and edoxaban) at supply point upstream the hemofilter and upstream the blood pump; a replacement fluid source for supplying a replacement fluid upstream the hemofilter and upstream the blood pump; a blood analyzer connected to receive blood samples from one or both of the withdrawn blood line and the return line.

Useful types of blood analyzer include also a coagulation analyzer that collects samples from one or both of the withdrawn blood line and the return line. The coagulation analyzer may be configured to obtain measurements of coagulation and transmit them to the anticoagulant control unit which may in turn modulate the rate of infusion of anticoagulant to maintain a target coagulation level, or to maintain a steady coagulation level.

### SUMMARY

An extracorporeal blood treatment apparatus according to the present invention comprises the technical features as defined in independent claim 1.

The present disclosure sets forth extracorporeal blood treatment apparatus, used for performing extracorporeal blood treatments, such as Therapeutic Plasma Exchange (TPE), hemofiltration (HF), hemodialysis ("HD"), hemodiafiltration ("HDF"), hemoperfusion, toxins adsorption, as well as the treatments performed during continuous renal replacement treatments ("CRRT") and also ECCO₂R.
Document US2022054724A1 discloses an extracorporeal blood treatment apparatus according to the preamble of claim 1.

Additional features and advantages are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the disclosed subject matter.

### BRIEF DESCRIPTION OF THE FIGURES

Figure 1 is a schematic view of an extracorporeal blood treatment apparatus according to a first embodiment of the present invention;
Figure 2 is a schematic view of an extracorporeal blood treatment apparatus according to a second embodiment of the present invention;
Figure 3 is a schematic view of an extracorporeal blood treatment apparatus according to a third embodiment of the present invention;
Figure 4 is another schematic view of an extracorporeal blood treatment apparatus according to a fourth embodiment of the present invention;
Figure 5 is another schematic view of an extracorporeal blood treatment apparatus according to a fifth embodiment of the present invention;
Figure 6 is a flowchart of a method for monitoring at least one substance S into a fluid F in one of the embodiments of figures 1, 2 or 3.

### DETAILED DESCRIPTION

In the following description, some embodiments of extracorporeal blood treatment apparatuses will be firstly described being suitable, or designed, principally (though not exclusively) for intensive care treatments.

Unless defined otherwise, all technical and scientific terms used have the same meaning as commonly understood by one of ordinary skill in the art.

With reference to the attached figures, the numeral 1 globally refers to an extracorporeal blood treatment apparatus which may be used for performing any kind of kidney failure therapy, such as Hemodialysis ("HD"), Hemofiltration ("HF"), Hemodiafiltration ("HDF") and in the context of continuous renal replacement therapies (CRRT) with or without anticoagulation, for example CRRT with or without systemic anticoagulation (e.g., heparin) / with or without regional anticoagulation (e.g., citrate).

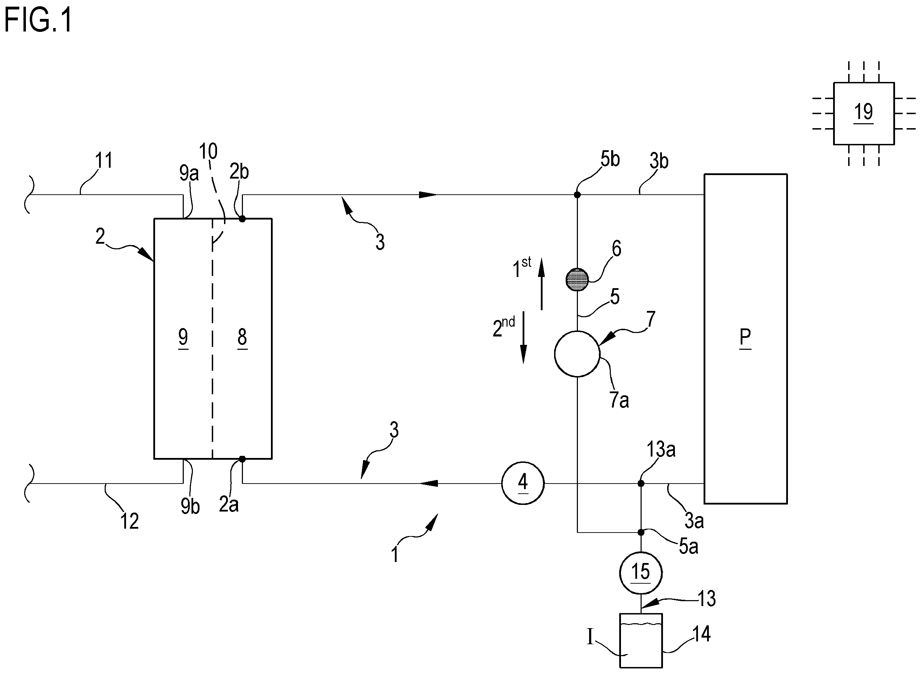

With reference to figures 1 to 5, the extracorporeal blood treatment apparatus 1 comprises a blood treatment unit 2, an extracorporeal blood circuit 3, comprising a blood withdrawal line 3a connected to the blood treatment unit 2, a blood return line 3b connected to the blood treatment unit 2, and a blood pump 4 active on the blood withdrawal line 3a. The blood withdrawal line 3a and the blood return line 3b are configured to be connected to a patient P.

The treatment unit 2 comprises a filtration unit of the type used for dialysis treatments and/or similar treatment.

In particular, the filtration unit has a primary chamber 8 and a secondary chamber 9 separated by a semi-permeable membrane 10. The blood withdrawal line 3a is connected to an inlet 2a of the primary chamber 8 of the filtration unit, whereas the blood return line 3b is connected to an outlet 2b of the primary chamber 8 of the filtration unit. Moreover, the extracorporeal blood treatment apparatus 1 is also provided with a dialysate line 11 connected to an inlet 9a of the secondary chamber 9 of the filtration unit and an effluent line 12 connected to an outlet 9b of the secondary chamber 9 of the filtration unit.

The extracorporeal blood treatment apparatus 1 of figure 1 also comprises a pre infusion line 13 connected to the blood withdrawal line 3a at a junction point 13a placed upstream the blood pump 4, to transfer a pre infusion solution I into blood flowing towards the filtration unit, a pre infusion fluid source 14 of the pre infusion solution I connected to the pre infusion line 13 and a pre infusion pump 15 active on the pre infusion line 13. In a variant embodiment, not shown, the blood pump 4 is located upstream of the junction point 13a, between the patient P and said junction point 13a.

The pre infusion pump 15 is drivable between an activated condition, wherein the pre infusion pump 15 causes the pre infusion solution I to flow from the corresponding pre infusion fluid source 14 towards the blood withdrawal line 3a, and an inactivated condition, wherein the pre infusion pump 15 is inactive and the pre infusion solution I does not flow from the corresponding pre infusion fluid source 14 through the corresponding pre infusion line 13. The pre infusion solution I supplied by the pre infusion fluid source may include a regional anticoagulant, such as citrate and/or citric acid.

The pre infusion pump 15 may be an occlusive pump (such as a peristaltic pump) that does not allow fluid to flow inside the line when it is stopped. Alternatively, or in combination, a clamp (or any other fluid blocking element) may be placed on the pre infusion line 13 to guarantee that no fluid flows inside the pre infusion line 13 when the pre infusion pump 15 is in the inactivated condition.

The pre infusion fluid source 14 may comprise at least one bag, optionally made of a plastic material. The bag of the infusion fluid source 14 may be disposable.

The extracorporeal blood treatment apparatus 1 also comprises a by-pass line 5 having a first junction end 5a in fluid communication with the blood withdrawal line 3a and a second junction end 5b in fluid communication with the blood return line 3b, a fluid flow regulator 7, for instance a by-pass pump 7a able to generate at least one fluid flow in the by-pass line 5, placed on the by-pass line 5, a sensor 6 placed on the by-pass line 5 for monitoring the concentration of at least one substance S in the blood or fluid flowing through the by-pass line 5, such as ionized calcium and/or magnesium. The by-pass pump 7a may be an occlusive pump and/or may be coupled to a by-pass valve, not shown, configured to prevent fluid or blood flow along the by-pass line 5.

In some embodiments, the by-pass line 5 with the sensor 6 and the by-pass pump 7a comprises or form part of a disposable portion or set connected or connectable to the extracorporeal blood circuit 3 through respective connectors. The disposable portion may be removably connected to the extracorporeal blood circuit 3.

In other embodiments, the by-pass line 5 with the sensor 6 and the by-pass pump 7a comprises a disposable portion connected to the blood circuit 3 and a fixed reusable (after sterilization) portion connected to a body of the extracorporeal blood treatment apparatus 1. The disposable portion and the fixed reusable portion being removably connected to the extracorporeal blood circuit 3.

The sensor 6 in the by-pass line 5 may be the only sensor configured to detect the at least one substance into any fluid flowing through the by-pass line 5, no other sensor for monitoring concentration being located on the extracorporeal blood circuit 3.

As shown in figure 1, the by-pass pump 7a is interposed between the 6 and the first junction end 5a of the by-pass line 5. It has to be noted that, in other embodiments, the by-pass pump 7a may also be interposed between the second junction end 5b and the sensor 6.

The sensor 6 may be any sensor suitable for detecting the concentration of the at least one substance S. The sensor 6 may be a non-invasive sensor (i.e., a sensor that does not come into contact with the blood). For example, the sensor 6 may comprise an optical sensor, such as a fluorescence optical sensor. In other embodiments, the sensor 6 may be of the kind requiring contact with blood/fluid, such as a Ion Selective Electrode sensor (ISE). The sensor 6 may also of the type requiring micro sampling. The sensor 6 on the by-pass line 5 may be one or more than one. If there are two or more sensors 6 on the by-pass line 5, these sensors 6 may be arranged in parallel or in series or in a combination of series and parallel. If there are two or more sensors 6 on the by-pass line 5, these sensors 6 may be configured to sense a same substance S or different substances.

The sensor 6 may also be configured to separately detect the concentration of two or more different substances S in sequence in different fluids that flow though the by-pass line 5. The sensor 6 may be configured to simultaneously detect the concentration of two different substances S into the same fluid flowing through the by-pass line 5.

The extracorporeal blood treatment apparatus 1 is also provided with a control unit 19 operatively connected to the by-pass pump 7a of the by-pass line 5 to control this latter between a first operating condition (i), wherein blood or fluid coming from the blood withdrawal line 3a flows through the by-pass line 5 to the sensor 6 and towards the blood return line 3b, and a second operating condition (ii), wherein blood coming from the blood return line 3b flows through the by-pass line 5 to the sensor 6 and towards the withdrawal line 3a.

The first operating condition (i) and the second operating condition (ii) may be repeated a plurality of times during an extracorporeal blood treatment, for instance according to a sufficient number of times to allow a proper monitoring of the patient P and/or the operation of the blood treatment apparatus 1 and eventually also to establish a trend over time of the measured data.

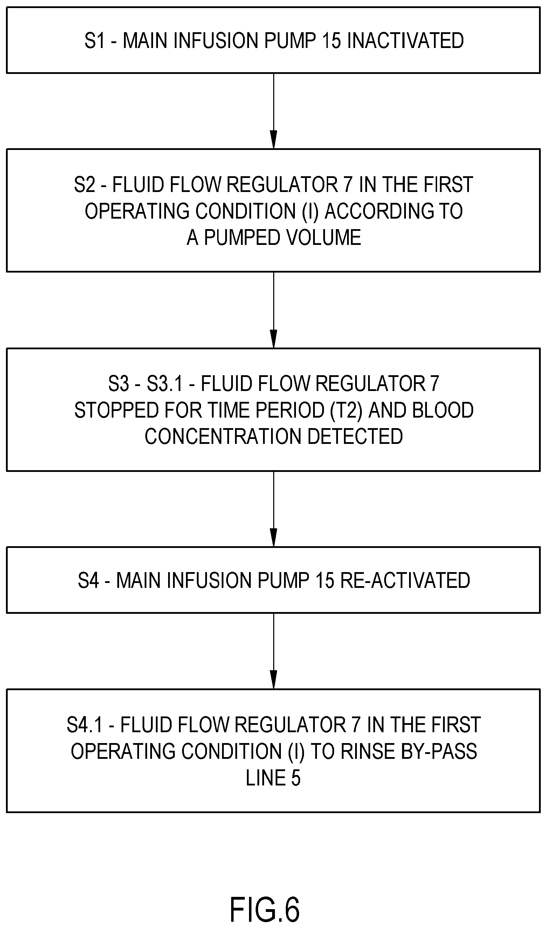

The control unit 19 may be the control unit of the apparatus 1 which is configured and programmed to manage all the operations of the apparatus 1. The control unit 19 is also connected to the blood pump 4 and to the pre infusion pump 15. The control unit 19 is also configured and programmed to carry out a monitoring method (MM) for monitoring at least one substance S into a fluid F, e.g. blood and/or infusion fluids. A schematic example of the method is shown in the flowchart of figure 6.

In the embodiment of figure 1, the first junction end 5a of the by-pass line 5 is directly connected to the pre infusion line 13, between the pre infusion pump 15 and the junction point 13a of the pre infusion line 13, and the second junction end 5b is directly connected to the blood return line 3b. Therefore, the by-pass line 5 may be put in fluid communication with the withdrawal line 3a through the pre infusion line 13.

In the first operating condition (i), blood coming from the blood withdrawal line 3a flows through the pre infusion line 13 and through the by-pass line 5 towards the sensor 6 and the return line 3b. In the second operating condition (ii), blood coming from the return line 3b flows through the by-pass line 5 to the sensor 6 and then towards the withdrawal line 3a through the pre infusion line 13.

In order to perform the monitoring operations through the sensor 6, the control unit 19 is further operatively connected to the sensor 6, so that control unit 19 is configured to receive one or more signals emitted by the sensor 6 to determine the concentration of the at least one substance S in the fluid flowing through the by-pass line 5.

For instance, in order to determine the concentration of the at least one substance S in the blood coming from the patient P and flowing in the blood withdrawal line 3a during an extracorporeal blood treatment, the control unit 19 maintains the pre infusion pump 15 in the inactivated condition, so that the pre infusion solution I does not flow downstream of the pre infusion pump 15, and drives the by-pass pump 7a in the first operating condition (i) to determine a blood flow through the by-pass line 5 from the first junction end 5a towards the second junction end 5b of this latter.

The patient's blood coming from the blood withdrawal line 3a of the extracorporeal blood circuit 3 flows through the pre infusion line 13, between the corresponding junction point 13a and the first junction end 5a of the by-pass line 5, flows into and along the by-pass line 5 towards the blood return line 5b of the extracorporeal blood circuit 3. In this situation, the sensor 6 may detect the concentration of the at least one substance S of the patient's blood, i.e. the systemic blood, running along the by-pass line 5 towards the blood return line 3b of the extracorporeal blood circuit 3.

In order to determine the concentration of the at least one substance S in the blood treated by the filtration unit and flowing in the blood return line 3b, the control unit 19 drives the by-pass pump 7a in the second operating condition (ii). The pre infusion pump 15 may keep running, so that no therapy interruption is required. The blood coming from the blood return line 3b flows through the by-pass line 5 to the sensor 6 towards the pre infusion line 13 and the blood withdrawal line 3a of the extracorporeal blood circuit 3. In this situation, the sensor 6 may detect the concentration of the at least one substance S of the blood treated by the filtration unit, running along the by-pass line 5.

For instance, the sensor 6 is configured to detect: urea, creatinine, uric acid, glucose, electrolytes, like Na, K, Ca, Mg, chloride, phosphates, lactate, bicarbonate.

In this second operating condition (ii), the control unit 19 may control the pre infusion pump 15 between the inactivated condition or the activated condition. If the pre infusion pump 15 is in the activated condition, then blood coming from the by-pass line 5 is diluted in the segment between the first junction end 5a and the junction point 13a of the pre infusion line 13 before reaching the withdrawal line 3a. If the pre infusion pump 15 is in the inactivated condition, the blood is not diluted in said segment.

In order to allow the sensor 6 to detect the at least one substance S in blood in the by-pass line 5, conditions of the blood in the by-pass line 5 depends on the type of sensor 6 employed. For instance, the sensor 6 may be configured to detect the at least one substance S while the blood is flowing in the by-pass line 5 or may require no-flow conditions to produce a measurement. In some embodiments, the sensor 6 may also comprise a sampling unit configured to take a blood sample and the at least one substance is detected independently of what happens afterwards in the by-pass line 5.

If the sensor 6 requires no-flow conditions, the by-pass pump 7a is driven in the first operating condition (i) or in the second operating condition (ii) according to a pumped volume (V) or according to a time lapse t1 sufficient to get a representative sample of the blood to the sensor 6. Then, the by-pass pump 7a is stopped and set in an inactive state or the by-pass valve is closed for a time period t2 which allows the sensor 6 to read a stable concentration value and detect the at least one substance. In this time period t2, the pre infusion pump 15 may be in the inactivated or in the activated condition, since the by-pass line 5 is closed and the extracorporeal blood treatment is running.

In order to get a representative blood sample at the sensor 6, in the first operating condition (i), the control unit 19 keeps the pre infusion pump 15 in the inactivated condition for a time sufficient to pump said pumped volume (V) or for said time lapse t1. The time lapse t1 is a function of the blood flow rate in the by-pass line 5, i.e. a flow rate determined by the by-pass pump 7a. The time lapse t1 is also function of a cross section of the by-pass line 5 and/or a volume and/or of a design of the by-pass line 5 between the first junction end 5a and the sensor 6 and/or of a distance of a circuit section defined between the junction point 13a and the sensor 6.

If the sensor 6 is configured to detect the at least one substance S while the blood is flowing in the by-pass line 5, the control unit 19 is configured to keep the by-pass pump 7a in the first operating condition (i) or in the second operating condition (ii) for a time required to allow the sensor 6 to read a stable concentrate value and to detect the at least one substance S.

Once that the at least one substance S has been detected, the by-pass pump 7a is driven again in the first operating condition (i), the pre infusion pump 15 is activated or kept active such that the pre infusion solution I flows along the by-pass line 5 towards the blood return line 3b and rinses the by-pass line 5, the by-pass pump 7a and the sensor 6. In order to fill the by-pass line 5 with the pre infusion solution I, the by-pass pump 7a may be operated at a flow rate less than the pre infusion pump 15. Rinsing allows to remove blood from the by-pass section 5 as to prevent clotting, blood losses and/or sensor drift further to excessive contact with blood.

This last configuration may also be used to start checking a composition of the pre infusion solution I through the sensor 6. The by-pass pump 7a is driven in the first operating condition (i) for a time sufficient to pump said pumped volume (V) or according to the time lapse t1 sufficient to get a representative sample of the pre infusion solution I to the sensor 6. Then, the by-pass pump 7a is stopped and set in an inactive state or the by-pass valve is closed for the time period t2 which allows the sensor 6 to read a stable concentration value or values and detect the substance or substances in the pre infusion solution I.

In some embodiments, the control unit 19 is also configured to adjust the amount of the pre infusion solution I supplied by the pre infusion fluid source 14 to be infused into the blood withdrawal line 3a as a function of the concentration of the at least one substance detected in blood by the sensor 6.

The extracorporeal blood treatment apparatus 1 comprises a user interface connected to the control unit 19 and comprising a screen for displaying data pertaining to the extracorporeal blood treatment apparatus 1 and the measured concentrations of the at least one substance S. For instance, the screen displays the measured concentrations of the at least one substance S, for example ionized calcium, versus anticoagulant dose, for example citrate dose.

In some embodiments, the control unit 19 is configured to compare the measured concentration of the at least one substance S and/or a trend of the measured concentration with a high threshold or thresholds and a low threshold or thresholds and to issue an alarm in case any threshold is crossed.

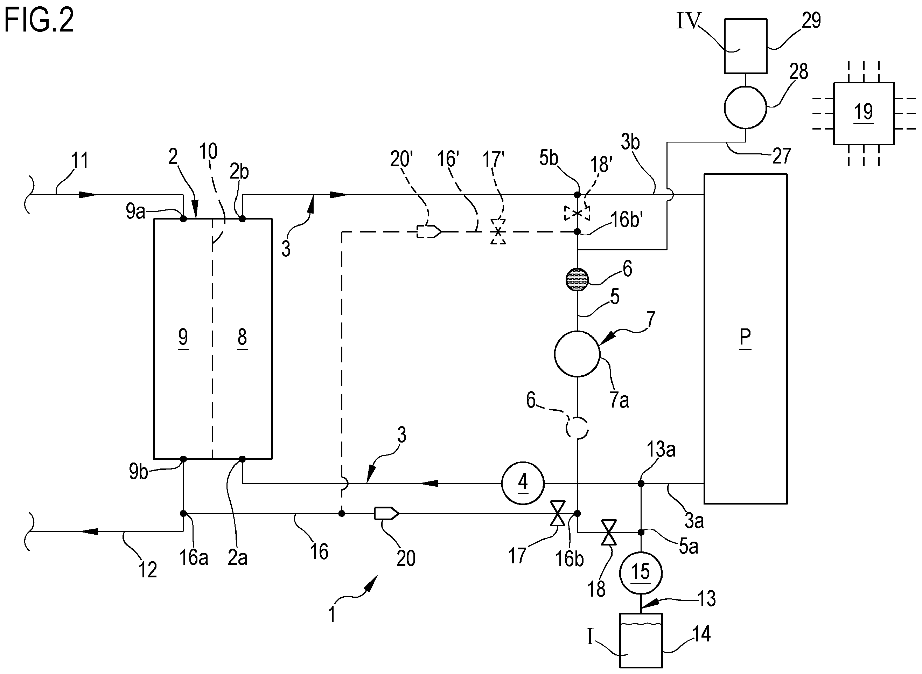

With reference to the embodiment shown in figure 2, the extracorporeal blood treatment apparatus 1 comprises the same elements of the embodiment of figure 1. In addition compared to the embodiment of figure 1, the apparatus 1 according to the embodiment of figure 2 comprises a connection bridge 16 developing between the effluent line 12 and the by-pass line 5. The connection bridge 16 has a first junction end 16a positioned at the effluent line 12 and a second junction end 16b positioned at the by-pass line 5.

The connection bridge 16 is provided with a filter 20 and one flow valve 17 operatively placed close to the second junction end 16b. The control unit 19 is operatively connected to the flow valve 17 to drive this latter between an open condition, in which the connection bridge 16 is in fluid communication with the by-pass line 5 and a closed condition, in which the connection bridge 16 is not in fluid communication with the by-pass line 5. The filter 20 is configured to prevent microbial contamination and may be a 0.2 µm filter.

The second junction end 16b of the connection bridge 16 represented in continuous line in figure 2 is interposed between the by-pass pump 7a and the first junction end 5a of the by-pass line 5. The by-pass line 5 is provided with a transit valve 18 interposed between the first junction end 5a of the by-pass line 5 and the second junction end 16b of the connection bridge 16. The control unit 19 is operatively connected to the transit valve 18 to drive this latter between an open condition, in which the by-pass line 5 is in fluid communication with the blood withdrawal line 3a through the pre infusion line 13, and a closed condition, in which the by-pass line 5 is not in fluid communication with the blood withdrawal line 3a.

In a variant embodiment, shown in dashed line in figure 2, the second junction end 16b' of the connection bridge 16' is interposed between the sensor 6 and the second junction end 5b of the by-pass line 5. In this case, the transit valve 18' is interposed between the second junction end 5b of the by-pass line 5 and the second junction end 16b' of the connection bridge 16'. The connection bridge 16' is provided with the filter 20' and the flow valve 17'. The control unit 19 is operatively connected to the transit valve 18' of the by-pass line 5 to drive this latter between an open condition, in which the by-pass line 5 is in fluid communication with the blood return line 3b and a closed condition, in which the by-pass line 5 is not in fluid communication with the blood return line 3b.

With reference to the embodiment in figure 2 provided with the connection bridge 16 represented in continuous line, the control unit 19 is configured to maintain the flow valve 17 in the open condition and the transit valve 18 in the closed condition to allow a flow of a fluid from the effluent line 12 towards the by-pass line 5. In this situation, the control unit 19 runs the by-pass pump 7a in the first operating condition in order to determine a flow of fluid from the second junction end 16b of the connection bridge 16 towards the blood return line 3b. When the by-pass pump 7a is in the first operating condition (i) the fluid coming from the effluent line 12 of the filtration unit flows through the connection bridge 16 into the by-pass line 5 to the sensor 6 and then towards the blood return line 3b.

With reference to the embodiment in figure 2 provided with the connection bridge 16 represented in dashed line, the control unit 19 is configured to maintain the flow valve 17 in the open condition and the transit valve 18 in the closed condition to allow the flow of a fluid from the effluent line 12 towards the by-pass line 5. In this situation, the control unit 19 runs the by-pass pump 7a in the second operating condition in order to determine a flow of fluid from the second junction end 16b of the connection bridge 16 towards the blood withdrawal line 3a through the pre infusion line 13. When the by-pass pump 7a is in the second operating condition, the fluid coming from the effluent line 12 of the filtration unit flows through the connection bridge 16 into the by-pass line 5 to the sensor 6 and then towards the blood withdrawal line 3a.

The sensor 6 may thus detect the at least one substance S into the fluid coming from the effluent line 12 and flowing through the by-pass line 5.

Referring to the embodiment in figure 2 with the connection bridge 16 in continuous line and similarly to what disclosed above with reference to detection in blood or in the pre infusion solution I, while the transit valve 18 is closed, the by-pass pump 7a is driven in the first operating condition (i) according to a pumped volume (V) or according to a time lapse t1 sufficient to get a representative sample of the effluent fluid to the sensor 6. Then, the flow valve 17 is closed and the by-pass pump 7a is stopped and set in an inactive state or the by-pass valve is closed for a time period t2 which allows the sensor 6 to read a stable concentration value and detect the at least one substance.

The time lapse t1 is a function of the effluent fluid flow rate in the by-pass line 5, i.e. a flow rate determined by the by-pass pump 7a. The time lapse t1 is also function of a cross section and/or design of the connection bridge 16 and of the by-pass line 5 between the second junction end 16b and the sensor 6.

Once that the at least one substance S in the effluent fluid has been detected, the by-pass pump 7a is driven again in the first operating condition (i) so that the effluent fluid flows into the blood return line or into a drain, not shown. Then, the transit valve 18 is opened and the pre infusion pump 15 is activated, such that the pre infusion solution I flows along the by-pass line 5 towards the return line 3b and rinses the by-pass line 5, the fluid flow regulator 7 and the sensor 6.

In other embodiments, depending on the type of sensor 6, the flow valve 17 is kept open and the by-pass pump 7a is running during measurement. Therefore, once that the at least one substance S in the effluent fluid has been detected, the flow valve 17 is closed and, if stopped before, the pre infusion pump 15 is re-activated.

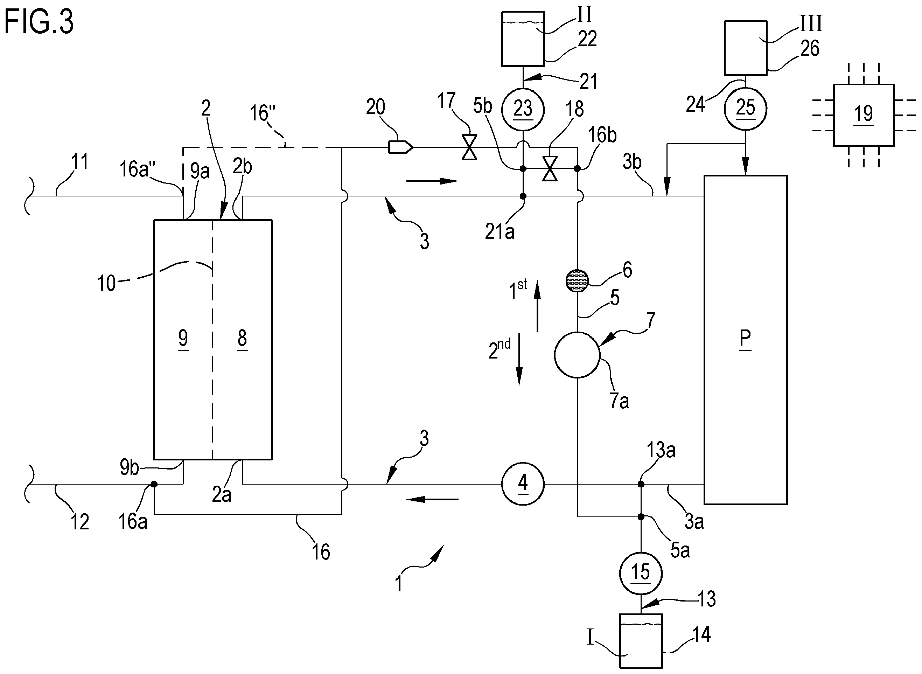

With reference to the embodiments shown in figure 3, the extracorporeal blood treatment apparatus 1 comprises the same elements of the embodiment of figure 1. In addition compared to the embodiment of figure 1, the apparatus 1 according to the embodiment of figure 3 further comprises a post infusion line 21 connected to the blood return line 3b at a connection point 21a to transfer a post infusion solution II into the treated blood coming from the filtration unit, a post infusion fluid source 22 of the post infusion solution II connected to the post infusion line 21 and a post infusion pump 23 active on the post infusion line 21.

The control unit 19 is configured to control the post infusion pump 23 between an activated condition, wherein the post infusion pump 23 causes the post infusion solution II to flow from the corresponding post infusion fluid source 22 to the blood return line 3b, and an inactivated condition, wherein the post infusion pump 23 is inactive and the post infusion solution II does not flow from the corresponding post infusion fluid source 22 through the corresponding post infusion line 21. The second junction end 5b of the by-pass line 5 directly connects to the post infusion line 21 between the connection point 21a and the post infusion pump 23 of the post infusion line 21 as may be seen in figure 3. The post infusion solution II comprises a substitute solution, for instance including at least one buffer agent, for instance bicarbonate.

The extracorporeal blood treatment apparatus 1 of figure 3 comprises the connection bridge 16 as in figure 2 provided with the filter 20 and the flow valve 17. The second junction end 16b of the connection bridge 16 is interposed between the sensor 6 and the second junction end 5b of the by-pass line 5 located on the post infusion line 21 and the sensor 6 is interposed between the by-pass pump 7a and the second junction end 16b. The transit valve 18 is located between the second junction end 5b of the by-pass line 5 and the second junction end 16b of the connection bridge 16.

The determination of the concentration of the at least one substance S in the blood coming from the patient P and flowing in the blood withdrawal line 3a may carried out as detailed with reference to the embodiment of figure 1 while the post infusion solution II is injected into the blood return line 3b through the post infusion pump 23 and the flow valve 17 is closed.

In order to determine the concentration of the at least one substance S in the blood coming from the filtration unit and flowing in the blood return line 3b, the control unit 19 maintains the post infusion pump 23 in the inactivated condition, so that the post infusion solution II does not flow downstream of the post infusion pump 23, and drives the by-pass pump 7a in the second operating condition (ii) to determine a blood flow through the by-pass line 5 from the connection point 21a, to the second junction end 5b and towards the first junction end 5a of this latter. Meanwhile, the pre infusion pump 15 may keep on infusing the pre infusion solution I in the blood withdrawal line 3a.

If the sensor 6 requires no-flow conditions, the by-pass pump 7a is driven in the second operating condition (ii) according a respective time lapse t1' and then the by-pass pump 7a is stopped and set in an inactive state or the by-pass valve is closed for the time period t2. If the sensor 6 is configured to detect the at least one substance S while the blood is flowing in the by-pass line 5, the control unit 19 is configured to keep the by-pass pump 7a in the second operating condition (ii) for the time required to allow the sensor 6 to read a stable concentrate value and to detect the at least one substance S.

Once that the at least one substance S has been detected, the by-pass pump 7a is driven in the second operating condition (ii) while the post infusion pump 23 is activated such that a fraction of the post infusion solution II flows along the by-pass line 5 towards the withdrawal line 3a and rinses the by-pass line 5, the by-pass pump 7a and the sensor 6. Otherwise, the by-pass pump 7a is driven in the first operating condition (ii) so that the pre infusion solution I flows along the by-pass line 5 towards the return line 3b and rinses the by-pass line 5, the by-pass pump 7a and the sensor 6.

A composition of the post infusion solution II may also be checked through the sensor 6. The by-pass pump 7a is driven in the second operating condition (ii) according to the pumped volume (V) or the time lapse t1 sufficient to get a representative sample of the post infusion solution II to the sensor 6. Then, the by-pass pump 7a is stopped and set in an inactive state or the by-pass valve is closed for the time period t2 which allows the sensor 6 to read a stable concentration value or values and detect the substance or substances in the post infusion solution II. In other embodiments, depending on the type of sensor 6, the by-pass pump 7a is running during measurement.

In some embodiments, the control unit 19 is also configured to adjust the amount of the post infusion solution II supplied by the post infusion fluid source 22 to be infused into the blood return line 3b on the base of the concentration of the at least one substance detected in blood by the sensor 6.

The sensor 6 may also detect the at least one substance S into the fluid coming from the effluent line 12. In order to carry out this detection, the control unit 19 maintains the flow valve 17 in the open condition and the transit valve 18 in the closed condition to allow a flow of a fluid from the effluent line 12 towards the by-pass line 5. In this situation, the control unit 19 runs the by-pass pump 7a in the second operating condition in order to determine a flow of fluid from the second junction end 16a of the connection bridge 16 towards the blood withdrawal line 3b.

In a variant embodiment, the connection bridge 16 or a further connection bridge 16", as shown in dashed line in figure 3, connects the dialysate line 11, from a respective first junction end 16a", to the by-pass line 5 and may be employed to check the composition of fluid in the dialysate line 11. The connection bridge 16 or the further connection bridge 16" may be connected to the by-pass line 5 as disclosed in figures 2 or 3 for the effluent line 12.

Figure 3 shows also an ion replenishment infusion line 24, an ion replenishment infusion pump 25 active on the ion replenishment infusion line 24 and an ion replenishment infusion source 26 of ion replenishment solution III connected to one end of the ion replenishment infusion line 24. The ion replenishment infusion line 24 may be connected to the blood return line 3b and/or directly into the patient P to infuse the ion replenishment solution III into the blood return line 3b and/or directly into the patient P. The ion replenishment solution (III) may comprise ionized calcium, for instance a concentrated solution of ionized calcium. The control unit 19 is configured to adjust the amount of the ion replenishment solution III supplied by the ion replenishment infusion source 26 on the base of the concentration of the at least one substance detected by the sensor 6 based on the concentration measured during the first operating condition (i) of the by-pass pump 7a.

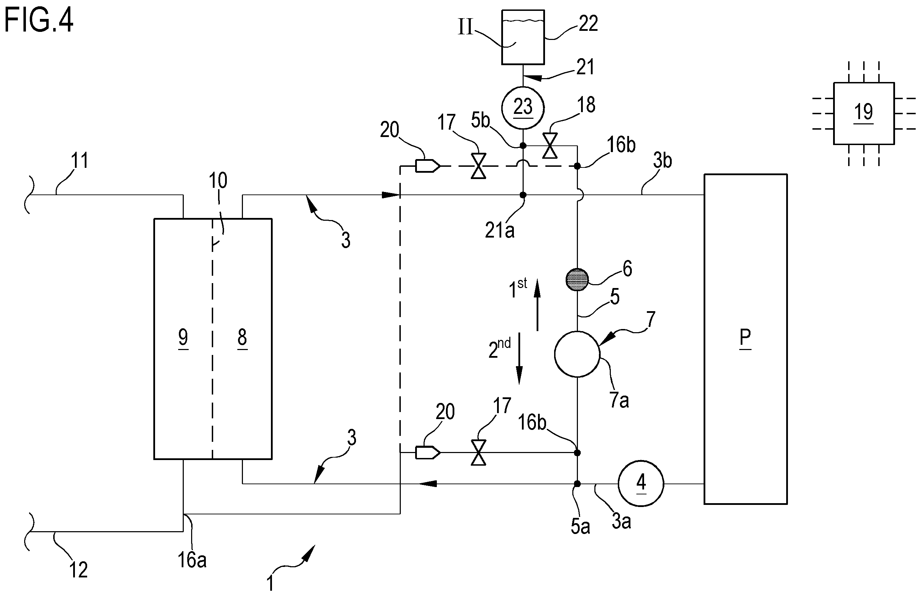

Figure 4 shows another embodiment in which the first junction end 5a is directly connected to with the blood withdrawal line 3a (not through the pre infusion line 13). The second junction end 5b of the by-pass line 5 directly connects to the post infusion line 21 between the connection point 21a and the post infusion pump 23 of the post infusion line 21, as in figure 3.

The second junction end 16b of the connection bridge 16 may interposed between the sensor 6 and the second junction end 5b of the by-pass line 5 located on the post infusion line 21 (dashed line) or may be connected to the by-pass line 5 between the by-pass pump 7a and the first junction end 5a (continuous line). The blood pump 4 is located upstream of the first junction end 5a, between the patient P and said first junction end 5a.

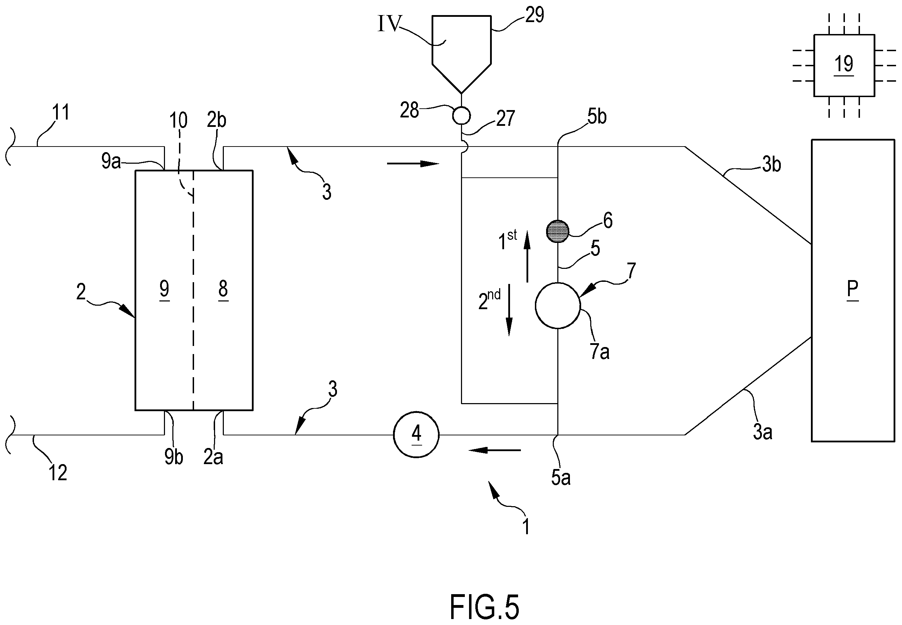

Figure 5 shows another basic embodiment in which the first junction end 5a is joined to the blood withdrawal line 3a and the second junction end 5b is joined to the blood return line 3b. This example embodiment comprises a calibration line 27 with a calibration pump 28 connected to a calibration source 29 of a calibration fluid IV. The calibration line 27 is also connected to the by-pass line 5. The control unit (19) is configured to drive the fluid flow regulator (7) according to the first operating condition (i) or according to the second operating condition (ii) to determine the calibration solution (IV) coming from the calibration source (29) to flow through the by-pass line (5) towards the return line (3b) or towards the withdrawal line (3a) in order to calibrate the sensor (6).

The calibration line 27 with a calibration pump 28 connected to a calibration source 29 may also be part of the other embodiments detailed above. For instance, as shown in Figure 2, the calibration line 27 is connected to the by-pass line 5 and, after calibration, the calibration fluid is discharged into the effluent line 12 through the connection bridge 16.

This embodiment may be provide with infusion lines, not shown, infusing directly in the extracorporeal blood circuit 3 or in the patient P. Anyway, these infusion lines are not connected to the by-pass line 5.

In all the embodiments herein disclosed, when detecting solutes in blood, the sensor (6) may be configured to detect concentration of a substance in blood that is not present in the infusion solution (I, II). For instance, the infusion solution I, II supplied by the infusion fluid source 14, 22 includes at least one solute that reacts with at least one component present in the blood, such as citrate reacting with ionized calcium that is present in the blood. For instance, when detecting solutes in blood, the sensor 6 may be configured to sense concentration of said component present in blood (e.g., ionized calcium).

The present monitoring method may be advantageously used for monitoring calcium in connection with cancer dialysis. Indeed, cancer-associated hypercalcemia (CAH) is the most frequent metabolic disorder in cancer patients. CAH is still associated with poor outcome. Hypercalcemia is a condition where there is too much calcium in the blood. It is the most common life-threatening complication of cancer in adults.

Hypercalcemia develops in adults with cancer and is a complication that develops in 10% to 20% of patients with cancer. Cancer-associated hypercalcemia often occurs late in the course of solid-tumor development and portends a poor prognosis. The management of hypercalcemia is based on the presence of characteristic symptomatology and the severity of calcium elevation. Patients with hypercalcemia of malignancy are typically symptomatic and have markedly elevated calcium levels.

Dialysis may have a fast impact on calcium reduction, and it remains a viable option to reduce hypercalcemia. Patients with acute hypercalcaemia may be considered for haemodialysis using a very low calcium dialysate (≤1 mmol/L), and monitoring of the blood calcium concentration with the concentration sensor of embodiments of the present description may offer significant advantages for feedback of therapy.

While the invention has been described in connection with what is presently considered to be the most practical and preferred embodiment, it is to be understood that the invention is not to be limited to the disclosed embodiment, but on the contrary, is intended to cover subject matter included within the scope of the appended claims.

## Claims

1. An extracorporeal blood treatment apparatus (1) comprising:
a blood treatment unit (2);
an extracorporeal blood circuit (3) comprising a blood withdrawal line (3a) connected to an inlet (2a) of the blood treatment unit (2), and a blood return line (3b) connected to an outlet (2b) of the blood treatment unit (2);
a blood pump (4) active on the blood withdrawal line (3a) of the extracorporeal blood circuit (3);
a by-pass line (5) having a first junction end (5a) in fluid communication with the blood withdrawal line (3a) of the extracorporeal blood circuit (3) and a second junction end (5b) in fluid communication with the blood return line (3b) of the extracorporeal blood circuit (3);
a sensor (6) placed on the by-pass line (5) for monitoring the concentration of at least one substance in the blood or fluid flowing through the by-pass line (5);
**characterized in that** the extracorporeal blood treatment apparatus (1) further comprises:
a fluid flow regulator (7) placed on the by-pass line (5); and
a control unit (19) operatively connected to the fluid flow regulator (7) to control this latter between:
i. a first operating condition, wherein blood or fluid coming from the blood withdrawal line (3a) flows through the by-pass line (5) towards the blood return line (3b);
ii. a second operating condition, wherein blood or fluid coming from the blood return line (3b) flows through the by-pass line (5) towards the withdrawal line (3a).

2. The apparatus (1) according to claim 1, further comprising:
an infusion line (13, 21) connected to the blood withdrawal line (3a) and/or to the blood return line (3b) at a point (13a, 21a);
an infusion fluid source (14, 22) connected to the infusion line (13, 21), the infusion fluid source (14, 22) supplying an infusion solution (I, II);
an infusion pump (15, 23) active on the infusion line (13, 21); the control unit (19) being configured to control the infusion pump (15, 23) between an activated condition, wherein the infusion pump (15, 23) causes the infusion solution (I, II) to flow from the corresponding infusion fluid source (14, 22) to the blood withdrawal line (3a) and/or to the blood return line (3b), and an inactivated condition, wherein the infusion pump (15, 23) is inactive and the infusion solution (I, II) does not flow from the corresponding infusion fluid source (14, 22) through the corresponding infusion line (13, 21);
wherein the first junction end (5a) and/or the second junction end (5b) of the by-pass line (5) is/are connected to the infusion line (13, 21) between the infusion pump (15, 23) and the point (13a, 21a).

3. The apparatus (1) according to claim 2, wherein:
in the first operating condition (i), the control unit (19) is configured to maintain the infusion pump (15, 23) in the inactivated condition or in an activated condition and to control the fluid flow regulator (7) to cause blood in the blood withdrawal line (3a) of the extracorporeal blood circuit (3) to flow along the by-pass line (5) to the sensor (6) and towards the blood return line (3b), the sensor (6) monitoring the concentration of the at least one substance (S) in the blood coming from the blood withdrawal line (3a);
in the second operating condition (ii), the control unit (19) is configured to maintain the infusion pump (15, 23) in the inactivated condition or in the activated condition and to control the fluid flow regulator (7) to cause the blood in the blood return line (3b) of the extracorporeal blood circuit (3) to flow along the by-pass line (5) to the sensor (6) and towards the blood withdrawal line (3a), the sensor (6) monitoring the concentration of the at least one substance (S) in the blood coming from the blood return line (3b).

4. The apparatus (1) according to claim 2 or 3, wherein in the activated condition of the infusion pump (15, 23), the control unit (19) is configured to drive the fluid flow regulator (7) according to the first operating condition (i) or according to the second operating condition (ii) to determine at least a fraction of the infusion solution (I, II) coming from the fluid solution source (14, 22) to flow through the by-pass line (5) towards the return line (3b) or towards the withdrawal line (3a) to rinse the by-pass line (5), the fluid flow regulator (7) and/or the sensor (6) or to check a composition of the infusion solution (I, II) through the sensor (6).

5. The apparatus (1) according to any claims 1 to 4, wherein the blood treatment unit (2) is a blood filtration unit having a primary chamber (8) and a secondary chamber (9) separated by a semi-permeable membrane (10), the blood withdrawal line (3a) being connected to the inlet (2a) of the primary chamber (8) and the blood return line (3b) being connected to the outlet (2b) of the primary chamber (8); the blood treatment unit (2) being also provided with an effluent line (12) connected to an outlet (9b) of the secondary chamber (9) and optionally of a dialysate line (11) connected to an inlet (9a) of the primary chamber (9); wherein the extracorporeal blood treatment apparatus (1) comprises a connection bridge (16, 16') developing between the effluent line (12), and/or the optional dialysate line (11), and the by-pass line (5), the connection bridge (16, 16') having a first junction end (16a) positioned at the effluent line (12), and/or at the optional dialysate line (11), and a second junction end (16b) positioned at the by-pass line (5); the connection bridge (16, 16') being provided with one flow valve (17); the control unit (19) being connected to the flow valve (17) of the connection bridge (16, 16') to drive this latter between an open condition, wherein the connection bridge (16, 16') is in fluid communication with the by-pass line (5) and a closed condition, wherein the connection bridge (16, 16') is not in fluid communication with the by-pass line (5), optionally the connection bridge (16, 16') being provided with a filter (20).

6. The apparatus (1) of claim 5, wherein the control unit (19) is configured to maintain the flow valve (17) in the open condition and the fluid flow regulator (7) in the first operating condition (i) or in the second operating condition (ii), such that a fluid coming from the effluent line (12), and/or at the optional dialysate line (11), flows through the connection bridge (16, 16') into the by-pass line (5) to the sensor (6) and towards the blood return line (3b) or towards the withdrawal line (3a), the sensor (6) detecting the at least one substance (S) into the fluid coming from the effluent line (12) and/or at the optional dialysate line (11) and flowing through the by-pass line (5).

7. The apparatus (1) of claim 5 or 6, wherein the by-pass line (5) is provided with at least one transit valve (18) interposed between the first junction end (5a) or the second junction end (5b) of the by-pass line (5) and the second junction end (16b) of the connection bridge (16, 16'), the control unit (19) being connected to the transit valve (18) of the by-pass line (5) to drive this latter between an open condition, wherein the by-pass line (5) is in fluid communication with the blood withdrawal line (3a) or with the blood return line (3b), and a closed condition, wherein the by-pass line (5) is not in fluid communication with the blood withdrawal line (3a) or with the blood return line (3b).

8. The apparatus (1) of any of claims 1 to 7, wherein the sensor (6) is located between the fluid flow regulator (7) and the first junction end (5a) or between the fluid flow regulator (7) and the second junction end (5b).

9. The apparatus (1) of claim 8 when depending on claim 5 or 6 or 7, wherein the sensor (6) is located between the fluid flow regulator (7) and the second junction end (16b) of the connection bridge (16, 16') or the fluid flow regulator (7) is located between the sensor (6) and the second junction end (16b) of the connection bridge (16, 16').

10. The apparatus (1) of claim 2, 3 or 4 or of any of claims 5 to 9 when depending on claim 2 or 3 or 4, wherein the infusion line (13, 21) comprises: a pre infusion line (13) connected to the blood withdrawal line (3a) at a junction point (13a);
wherein the infusion source (14, 22) comprises: a pre infusion fluid source (14) of a pre infusion solution (I) connected to the pre infusion line (13);
wherein the infusion pump (15, 23) comprises: a pre infusion pump (15) active on the pre infusion line (13), the control unit (19) being configured to control the pre infusion pump (15) between the activated condition, wherein the pre infusion pump (15) causes the pre infusion solution (I) to flow from the corresponding pre infusion fluid source (14) to the blood withdrawal line (3a) of the extracorporeal blood circuit (3), and the inactivated condition, wherein the pre infusion pump (15) is inactive and the pre infusion solution (I) does not flow from the corresponding pre infusion fluid source (14) through the corresponding pre infusion line (13), wherein the first junction end (5a) of the by-pass line (5) directly connects to the pre infusion line (13) between the junction point (13a) and the pre infusion pump (15) of the pre infusion line (13).

11. The apparatus (1) of claim 10 when depending on claim 5 or 6 or 7, wherein the second junction end (16b) of the connection bridge (16) is interposed between the sensor (6) and the first junction end (5a) of the by-pass line (5); optionally the second junction end (16b) of the connection bridge (16) being interposed between the fluid flow regulator (7) of the by-pass line (5) and the first junction end (5a) of the by-pass line (5) and the sensor (6) being interposed between the fluid flow regulator (7) of the by-pass line (5) and the second junction end (5b) to the by-pass line (5).

12. The apparatus (1) of claim 2, 3, 4, 10 or 11 or of any of claims 5 to 9 when depending on claim 2 or 3 or 4, wherein the infusion line (13, 21) comprises: a post infusion line (21) connected to the blood return line (3b) at a connection point (21a);
wherein the infusion source (14, 22) comprises: a post infusion fluid source (22) of a post infusion solution (II) connected to the post infusion line (21);
wherein the infusion pump (15, 23) comprises: a post infusion pump (23) active on the post infusion line (21), the control unit (19) being configured to control the post infusion pump (23) between the activated condition, wherein the post infusion pump (23) causes the post infusion solution (II) to flow from the corresponding post infusion fluid source (22) to the blood return line (3b) of the extracorporeal blood circuit (3), and the inactivated condition, wherein the post infusion pump (23) is inactive and the post infusion solution (II) does not flow from the corresponding post infusion fluid source (22) through the corresponding post infusion line (21), wherein the second junction end (5b) of the by-pass line (5) directly connects to the post infusion line (21) between the connection point (21a) and the post infusion pump (23) of the post infusion line (21).

13. The apparatus (1) of claim 12 when depending on claim 10 or 11, wherein:
in the first operating condition (i), the control unit (19) is configured to maintain the pre infusion pump (15) in the inactivated condition, the post infusion pump (23) in the activated condition or the inactivated condition and to control the fluid flow regulator (7) to cause blood in the blood withdrawal line (3a) of the extracorporeal blood circuit (3) to flow through the pre infusion line (13), along the by-pass line (5) to the sensor (6) and towards the blood return line (3b) through the post infusion line (21), the sensor (6) monitoring the concentration of the at least one substance (S) in the blood coming from the blood withdrawal line (3a);
in the second operating condition (ii), the control unit (19) is configured to maintain the pre infusion pump (15) in the inactivated or the activated condition, the post infusion pump (23) in the inactivated condition and to control the fluid flow regulator (7) to cause the blood in the blood return line (3b) of the extracorporeal blood circuit (3) to flow through the post infusion line (21), along the by-pass line (5) to the sensor (6) and towards the blood withdrawal line (3a) through the pre infusion line (13), the sensor (6) monitoring the concentration of the at least one substance (S) in the blood coming from the blood return line (3b).

14. The apparatus (1) according to any of claims 1 to 13, wherein the fluid flow regulator (7) comprises a by-pass pump (7a) active on the by-pass line (5) to generate at least one fluid flow in the by-pass line (5) at least according to the first operating condition (i) and the second operating condition (ii).

15. The apparatus (1) according to claim 14, wherein the by-pass pump (7a) is an occlusive pump and/or at least one by-pass valve is coupled to the by-pass pump (7a), is operative on the by-pass line (5) and is controlled by the control unit (19).

## Patentansprüche

1. Extrakorporales Blutbehandlungsgerät (1), umfassend:
eine Blutbehandlungseinheit (2);
einen extrakorporalen Blutkreislauf (3), umfassend eine Blutentnahmeleitung (3a), die mit einem Einlass (2a) der Blutbehandlungseinheit (2) verbunden ist, und eine Blutrückgabeleitung (3b), die mit einem Auslass (2b) der Blutbehandlungseinheit (2) verbunden ist;
eine Blutpumpe (4), die an der Blutentnahmeleitung (3a) des extrakorporalen Blutkreislaufs (3) aktiv ist;
eine Umgehungsleitung (5) mit einem ersten Anschlussende (5a) in Fluidkommunikation mit der Blutentnahmeleitung (3a) des extrakorporalen Blutkreislaufs (3) und einem zweiten Anschlussende (5b) in Fluidkommunikation mit der Blutrückgabeleitung (3b) des extrakorporalen Blutkreislaufs (3);
einen Sensor (6), der an der Umgehungsleitung (5) platziert ist, um die Konzentration mindestens einer Substanz in dem Blut oder Fluid, das durch die Umgehungsleitung (5) fließt, zu überwachen;
**dadurch gekennzeichnet, dass** das extrakorporale Blutbehandlungsgerät (1) ferner umfasst:
einen Fluidflussregler (7), der an der Umgehungsleitung (5) platziert ist; und
eine Steuereinheit (19), die mit dem Fluidflussregler (7) operativ verbunden ist, um letzteren zu steuern zwischen:
i. einem ersten Betriebszustand, bei dem Blut oder Fluid, das von der Blutentnahmeleitung (3a) kommt, durch die Umgehungsleitung (5) in Richtung der Blutrückgabeleitung (3b) fließt;
ii. einem zweiten Betriebszustand, bei dem aus der Blutrückgabeleitung (3b) kommendes Blut oder Fluid durch die Umgehungsleitung (5) in Richtung der Entnahmeleitung (3a) fließt.

2. Gerät (1) nach Anspruch 1, ferner umfassend:
eine Infusionsleitung (13, 21), die an einem Punkt (13a, 21a) mit der Blutentnahmeleitung (3a) und/oder der Blutrückgabeleitung (3b) verbunden ist;
eine Infusionsfluidquelle (14, 22), die mit der Infusionsleitung (13, 21) verbunden ist, wobei die Infusionsfluidquelle (14, 22) eine Infusionslösung (I, II) zuführt;
eine Infusionspumpe (15, 23), die an der Infusionsleitung (13, 21) aktiv ist; wobei die Steuereinheit (19) dazu ausgelegt ist, die Infusionspumpe (15, 23) zwischen einem aktivierten Zustand, bei dem die Infusionspumpe (15, 23) bewirkt, dass die Infusionslösung (I, II) von der entsprechenden Infusionsfluidquelle (14, 22) zu der Blutentnahmeleitung (3a) und/oder zu der Blutrückgabeleitung (3b) fließt, und einem inaktivierten Zustand zu steuern, bei dem die Infusionspumpe (15, 23) inaktiv ist und die Infusionslösung (I, II) nicht von der entsprechenden Infusionsfluidquelle (14, 22) durch die entsprechende Infusionsleitung (13, 21) fließt;
wobei das erste Anschlussende (5a) und/oder das zweite Anschlussende (5b) der Umgehungsleitung (5) zwischen der Infusionspumpe (15, 23) und dem Punkt (13a, 21a) mit der Infusionsleitung (13, 21) verbunden ist/sind.

3. Gerät (1) nach Anspruch 2, wobei:
in dem ersten Betriebszustand (i) die Steuereinheit (19) dazu ausgelegt ist, die Infusionspumpe (15, 23) in dem inaktivierten Zustand oder in einem aktivierten Zustand zu halten und den Fluidflussregler (7) zu steuern, um zu bewirken, dass Blut in der Blutentnahmeleitung (3a) des extrakorporalen Blutkreislaufs (3) entlang der Umgehungsleitung (5) zu dem Sensor (6) und in Richtung der Blutrückgabeleitung (3b) fließt, wobei der Sensor (6) die Konzentration der mindestens einen Substanz (S) in dem aus der Blutentnahmeleitung (3a) kommenden Blut überwacht;
in dem zweiten Betriebszustand (ii) die Steuereinheit (19) dazu ausgelegt ist, die Infusionspumpe (15, 23) in dem inaktivierten Zustand oder in dem aktivierten Zustand zu halten und den Fluidflussregler (7) zu steuern, um zu bewirken, dass das Blut in der Blutrückgabeleitung (3b) des extrakorporalen Blutkreislaufs (3) entlang der Umgehungsleitung (5) zu dem Sensor (6) und in Richtung der Blutentnahmeleitung (3a) fließt, wobei der Sensor (6) die Konzentration der mindestens einen Substanz (S) in dem von der Blutrückgabeleitung (3b) kommenden Blut überwacht.

4. Gerät (1) nach Anspruch 2 oder 3, wobei die Steuereinheit (19) dazu ausgelegt ist, in dem aktivierten Zustand der Infusionspumpe (15, 23) den Fluidflussregler (7) gemäß dem ersten Betriebszustand (i) oder gemäß dem zweiten Betriebszustand (ii) anzutreiben, um zu bestimmen, dass mindestens ein Anteil der Infusionslösung (I, II), der von der Fluidlösungsquelle (14, 22) kommt, durch die Umgehungsleitung (5) in Richtung der Rückgabeleitung (3b) oder in Richtung der Entnahmeleitung (3a) fließt, um die Umgehungsleitung (5), den Fluidflussregler (7) und/oder den Sensor (6) zu spülen, oder um eine Zusammensetzung der Infusionslösung (I, II) durch den Sensor (6) zu überprüfen.

5. Gerät (1) nach einem der Ansprüche 1 bis 4, wobei die Blutbehandlungseinheit (2) eine Blutfiltrationseinheit mit einer Primärkammer (8) und einer Sekundärkammer (9) ist, die durch eine semipermeable Membran (10) getrennt sind, wobei die Blutentnahmeleitung (3a) mit dem Einlass (2a) der Primärkammer (8) verbunden ist und die Blutrückgabeleitung (3b) mit dem Auslass (2b) der Primärkammer (8) verbunden ist; wobei die Blutbehandlungseinheit (2) ferner mit einer Abflussleitung (12), die mit einem Auslass (9b) der Sekundärkammer (9) verbunden ist, und gegebenenfalls einer Dialysatleitung (11) ausgestattet ist, die mit einem Einlass (9a) der Primärkammer (9) verbunden ist; wobei das extrakorporale Blutbehandlungsgerät (1) eine Verbindungsbrücke (16, 16') umfasst, die sich zwischen der Abflussleitung (12) und/oder der optionalen Dialysatleitung (11) und der Umgehungsleitung (5) erstreckt, wobei die Verbindungsbrücke (16, 16') ein erstes Anschlussende (16a), das an der Abflussleitung (12) und/oder an der optionalen Dialysatleitung (11) positioniert ist, und ein zweites Anschlussende (16b) aufweist, das an der Umgehungsleitung (5) positioniert ist; wobei die Verbindungsbrücke (16, 16') mit einem Flussventil (17) ausgestattet ist; wobei die Steuereinheit (19) mit dem Flussventil (17) der Verbindungsbrücke (16, 16') verbunden ist, um letzteres zwischen einem offenen Zustand, bei dem die Verbindungsbrücke (16, 16') in Fluidkommunikation mit der Umgehungsleitung (5) ist, und einem geschlossenen Zustand anzutreiben, bei dem die Verbindungsbrücke (16, 16') nicht in Fluidkommunikation mit der Umgehungsleitung (5) ist, wobei die Verbindungsbrücke (16, 16') gegebenenfalls mit einem Filter (20) ausgestattet ist.

6. Gerät (1) nach Anspruch 5, wobei die Steuereinheit (19) dazu ausgelegt ist, das Flussventil (17) in dem offenen Zustand zu halten und den Fluidflussregler (7) in dem ersten Betriebszustand (i) oder in dem zweiten Betriebszustand (ii) zu halten, so dass ein Fluid, das von der Abflussleitung (12) kommt und/oder an der optionalen Dialysatleitung (11) ist, durch die Verbindungsbrücke (16, 16') in die Umgehungsleitung (5) zu dem Sensor (6) und in Richtung der Blutrückgabeleitung (3b) oder in Richtung der Entnahmeleitung (3a) fließt, wobei der Sensor (6) die mindestens eine Substanz (S) in dem Fluid, das von der Abflussleitung (12) kommt, und/oder an der optionalen Dialysatleitung (11) ist und durch die Umgehungsleitung (5) fließt, detektiert.

7. Gerät (1) nach Anspruch 5 oder 6, wobei die Umgehungsleitung (5) mit mindestens einem Durchgangsventil (18) versehen ist, das zwischen dem ersten Anschlussende (5a) oder dem zweiten Anschlussende (5b) der Umgehungsleitung (5) und dem zweiten Anschlussende (16b) der Verbindungsbrücke (16, 16') angeordnet ist, wobei die Steuereinheit (19) mit dem Durchgangsventil (18) der Umgehungsleitung (5) verbunden ist, um letzteres zwischen einem offenen Zustand, bei dem die Umgehungsleitung (5) in Fluidkommunikation mit der Blutentnahmeleitung (3a) oder mit der Blutrückgabeleitung (3b) ist, und einem geschlossenen Zustand anzutreiben, bei dem die Umgehungsleitung (5) nicht in Fluidkommunikation mit der Blutentnahmeleitung (3a) oder mit der Blutrückgabeleitung (3b) ist.

8. Gerät (1) nach einem der Ansprüche 1 bis 7, wobei sich der Sensor (6) zwischen dem Fluidflussregler (7) und dem ersten Anschlussende (5a) oder zwischen dem Fluidflussregler (7) und dem zweiten Anschlussende (5b) befindet.

9. Gerät (1) nach Anspruch 8, wenn abhängig von Anspruch 5 oder 6 oder 7, wobei sich der Sensor (6) zwischen dem Fluidflussregler (7) und dem zweiten Anschlussende (16b) der Verbindungsbrücke (16, 16') befindet oder sich der Fluidflussregler (7) zwischen dem Sensor (6) und dem zweiten Anschlussende (16b) der Verbindungsbrücke (16, 16') befindet.

10. Gerät (1) nach Anspruch 2, 3 oder 4 oder einem der Ansprüche 5 bis 9, wenn abhängig von Anspruch 2 oder 3 oder 4, wobei die Infusionsleitung (13, 21) umfasst: eine Vorinfusionsleitung (13), die an einem Anschlusspunkt (13a) mit der Blutentnahmeleitung (3a) verbunden ist; wobei die Infusionsquelle (14, 22) umfasst: eine Vorinfusionsfluidquelle (14) einer Vorinfusionslösung (I), die mit der Vorinfusionsleitung (13) verbunden ist; wobei die Infusionspumpe (15, 23) umfasst: eine Vorinfusionspumpe (15), die an der Vorinfusionsleitung (13) aktiv ist, wobei die Steuereinheit (19) dazu ausgelegt ist, die Vorinfusionspumpe (15) zwischen dem aktivierten Zustand, bei dem die Vorinfusionspumpe (15) bewirkt, dass die Vorinfusionslösung (I) von der entsprechenden Vorinfusionsfluidquelle (14) zu der Blutentnahmeleitung (3a) des extrakorporalen Blutkreislaufs (3) fließt, und dem inaktivierten Zustand zu steuern, bei dem die Vorinfusionspumpe (15) inaktiv ist und die Vorinfusionslösung (I) nicht von der entsprechenden Vorinfusionsfluidquelle (14) durch die entsprechende Vorinfusionsleitung (13) fließt, wobei das erste Anschlussende (5a) der Umgehungsleitung (5) direkt mit der Vorinfusionsleitung (13) zwischen dem Anschlusspunkt (13a) und der Vorinfusionspumpe (15) der Vorinfusionsleitung (13) verbunden ist.

11. Gerät (1) nach Anspruch 10, wenn abhängig von Anspruch 5 oder 6 oder 7, wobei das zweite Anschlussende (16b) der Verbindungsbrücke (16) zwischen dem Sensor (6) und dem ersten Anschlussende (5a) der Umgehungsleitung (5) angeordnet ist; wobei gegebenenfalls das zweite Anschlussende (16b) der Verbindungsbrücke (16) zwischen dem Fluidflussregler (7) der Umgehungsleitung (5) und dem ersten Anschlussende (5a) der Umgehungsleitung (5) angeordnet ist, und der Sensor (6) zwischen dem Fluidflussregler (7) der Umgehungsleitung (5) und dem zweiten Anschlussende (5b) zur Umgehungsleitung (5) angeordnet ist.

12. Gerät (1) nach Anspruch 2, 3, 4, 10 oder 11 oder nach einem der Ansprüche 5 bis 9, wenn abhängig von Anspruch 2 oder 3 oder 4, wobei die Infusionsleitung (13, 21) umfasst: eine Nachinfusionsleitung (21), die an einem Verbindungspunkt (21a) mit der Blutrückgabeleitung (3b) verbunden ist;
wobei die Infusionsquelle (14, 22) umfasst: eine Nachinfusionsfluidquelle (22) einer Nachinfusionslösung (II), die mit der Nachinfusionsleitung (21) verbunden ist;
wobei die Infusionspumpe (15, 23) umfasst: eine Nachinfusionspumpe (23), die an der Nachinfusionsleitung (21) aktiv ist, wobei die Steuereinheit (19) dazu ausgelegt ist, die Nachinfusionspumpe (23) zwischen dem aktivierten Zustand, bei dem die Nachinfusionspumpe (23) bewirkt, dass die Nachinfusionslösung (II) von der entsprechenden Nachinfusionsfluidquelle (22) zu der Blutrückgabeleitung (3b) des extrakorporalen Blutkreislaufs (3) fließt, und dem inaktivierten Zustand zu steuern, bei dem die Nachinfusionspumpe (23) inaktiv ist und die Nachinfusionslösung (II) nicht von der entsprechenden Nachinfusionsfluidquelle (22) durch die entsprechende Nachinfusionsleitung (21) fließt, wobei das zweite Anschlussende (5b) der Umgehungsleitung (5) zwischen dem Verbindungspunkt (21a) und der Nachinfusionspumpe (23) der Nachinfusionsleitung (21) direkt mit der Nachinfusionsleitung (21) verbunden ist.

13. Gerät (1) nach Anspruch 12, wenn abhängig von Anspruch 10 oder 11, wobei:
in dem ersten Betriebszustand (i) die Steuereinheit (19) dazu ausgelegt ist, die Vorinfusionspumpe (15) in dem inaktivierten Zustand zu halten, die Nachinfusionspumpe (23) in dem aktivierten Zustand oder dem inaktivierten Zustand zu halten und den Fluidflussregler (7) zu steuern, um zu bewirken, dass Blut in der Blutentnahmeleitung (3a) des extrakorporalen Blutkreislaufs (3) durch die Vorinfusionsleitung (13) entlang der Umgehungsleitung (5) zu dem Sensor (6) und in Richtung der Blutrückgabeleitung (3b) durch die Nachinfusionsleitung (21) fließt, wobei der Sensor (6) die Konzentration der mindestens einen Substanz (S) in dem von der Blutentnahmeleitung (3a) kommenden Blut überwacht;
in dem zweiten Betriebszustand (ii) die Steuereinheit (19) dazu ausgelegt ist, die Vorinfusionspumpe (15) in dem inaktivierten oder dem aktivierten Zustand zu halten, die Nachinfusionspumpe (23) in dem inaktivierten Zustand zu halten und den Fluidflussregler (7) zu steuern, um zu bewirken, dass das Blut in der Blutrückgabeleitung (3b) des extrakorporalen Blutkreislaufs (3) durch die Nachinfusionsleitung (21) entlang der Umgehungsleitung (5) zu dem Sensor (6) und durch die Vorinfusionsleitung (13) in Richtung der Blutentnahmeleitung (3a) fließt, wobei der Sensor (6) die Konzentration der mindestens einen Substanz (S) in dem von der Blutrückgabeleitung (3b) kommenden Blut überwacht.

14. Gerät (1) nach einem der Ansprüche 1 bis 13, wobei der Fluidflussregler (7) eine Umgehungspumpe (7a) umfasst, die auf der Umgehungsleitung (5) aktiv ist, um mindestens einen Fluidfluss in der Umgehungsleitung (5) mindestens gemäß dem ersten Betriebszustand (i) und dem zweiten Betriebszustand (ii) zu erzeugen.

15. Gerät (1) nach Anspruch 14, wobei die Umgehungspumpe (7a) eine okklusive Pumpe ist und/oder mindestens ein Umgehungsventil mit der Umgehungspumpe (7a) gekoppelt ist, an der Umgehungsleitung (5) wirksam ist und durch die Steuereinheit (19) gesteuert wird.

## Revendications

1. Appareil de traitement sanguin extracorporel (1) comprenant :
une unité de traitement sanguin (2) ;
un circuit sanguin extracorporel (3) comprenant un conduit de prélèvement de sang (3a) relié à une entrée (2a) de l'unité de traitement sanguin (2), et un conduit de retour de sang (3b) relié à une sortie (2b) de l'unité de traitement sanguin (2) ;
une pompe à sang (4) active sur le conduit de prélèvement de sang (3a) du circuit sanguin extracorporel (3) ;
un conduit de dérivation (5) ayant une première extrémité de jonction (5a) en communication fluidique avec le conduit de prélèvement de sang (3a) du circuit sanguin extracorporel (3) et une seconde extrémité de jonction (5b) en communication fluidique avec le conduit de retour de sang (3b) du circuit sanguin extracorporel (3) ;
un capteur (6) placé sur le conduit de dérivation (5) pour surveiller la concentration d'au moins une substance dans le sang ou le fluide s'écoulant à travers le conduit de dérivation (5) ;
**caractérisé en ce que** l'appareil de traitement sanguin extracorporel (1) comprend en outre :
un régulateur de débit de fluide (7) placé sur le conduit de dérivation (5) ; et
une unité de commande (19) reliée fonctionnellement au régulateur de débit de fluide (7) pour commander ce dernier entre :
i. une première condition de fonctionnement, du sang ou du fluide provenant du conduit de prélèvement de sang (3a) s'écoulant à travers le conduit de dérivation (5) vers le conduit de retour de sang (3b) ;
ii. une seconde condition de fonctionnement, du sang ou du fluide provenant du conduit de retour de sang (3b) s'écoulant à travers le conduit de dérivation (5) vers le conduit de prélèvement (3a).

2. Appareil (1) selon la revendication 1, comprenant en outre :
un conduit de perfusion (13, 21) relié au conduit de prélèvement de sang (3a) et/ou au conduit de retour de sang (3b) en un point (13a, 21a) ;
une source de fluide de perfusion (14, 22) reliée au conduit de perfusion (13, 21), la source de fluide de perfusion (14, 22) fournissant une solution de perfusion (I, II) ;
une pompe de perfusion (15, 23) active sur le conduit de perfusion (13, 21) ; l'unité de commande (19) étant configurée pour commander la pompe de perfusion (15, 23) entre une condition activée, la pompe de perfusion (15, 23) amenant la solution de perfusion (I, II) à s'écouler de la source de fluide de perfusion correspondante (14, 22) vers le conduit de prélèvement de sang (3a) et/ou vers le conduit de retour de sang (3b), et une condition inactivée, la pompe de perfusion (15, 23) étant inactive et la solution de perfusion (I, II) ne s'écoulant pas depuis la source de fluide de perfusion correspondante (14, 22) à travers le conduit de perfusion correspondant (13, 21) ;
la première extrémité de jonction (5a) et/ou la seconde extrémité de jonction (5b) du conduit de dérivation (5) étant reliée(s) au conduit de perfusion (13, 21) entre la pompe de perfusion (15, 23) et le point (13a, 21a).

3. Appareil (1) selon la revendication 2,
dans la première condition de fonctionnement (i), l'unité de commande (19) étant configurée pour maintenir la pompe de perfusion (15, 23) dans la condition inactivée ou dans une condition activée et à commander le régulateur de débit de fluide (7) afin d'amener le sang dans le conduit de prélèvement de sang (3a) du circuit sanguin extracorporel (3) à s'écouler le long du conduit de dérivation (5) vers le capteur (6) et vers le conduit de retour de sang (3b), le capteur (6) surveillant la concentration de l'au moins une substance (S) dans le sang provenant du conduit de prélèvement de sang (3a) ; dans la seconde condition de fonctionnement (ii), l'unité de commande (19) étant configurée pour maintenir la pompe de perfusion (15, 23) dans la condition inactivée ou dans la condition activée et à commander le régulateur de débit de fluide (7) afin d'amener le sang dans le conduit de retour de sang (3b) du circuit sanguin extracorporel (3) à s'écouler le long du conduit de dérivation (5) vers le capteur (6) et vers le conduit de prélèvement de sang (3a), le capteur (6) surveillant la concentration de l'au moins une substance (S) dans le sang provenant du conduit de retour de sang (3b).

4. Appareil (1) selon la revendication 2 ou 3, dans la condition activée de la pompe de perfusion (15, 23), l'unité de commande (19) étant configurée pour piloter le régulateur de débit de fluide (7) selon la première condition de fonctionnement (i) ou selon la seconde condition de fonctionnement (ii) pour amener au moins une fraction de la solution de perfusion (I, II) provenant de la source de solution de fluide (14, 22) à s'écouler à travers le conduit de dérivation (5) vers le conduit de retour (3b) ou vers le conduit de prélèvement (3a) pour rincer le conduit de dérivation (5), le régulateur de débit de fluide (7) et/ou le capteur (6) ou pour contrôler une composition de la solution de perfusion (I, II) par l'intermédiaire du capteur (6).

5. Appareil (1) selon l'une quelconque des revendications 1 à 4, l'unité de traitement sanguin (2) étant une unité de filtration du sang ayant une chambre primaire (8) et une chambre secondaire (9) séparées par une membrane semi-perméable (10), le conduit de prélèvement de sang (3a) étant relié à l'entrée (2a) de la chambre primaire (8) et le conduit de retour de sang (3b) étant relié à la sortie (2b) de la chambre primaire (8) ; l'unité de traitement sanguin (2) étant également pourvue d'un conduit d'effluent (12) relié à une sortie (9b) de la chambre secondaire (9) et facultativement d'un conduit de dialysat (11) relié à une entrée (9a) de la chambre primaire (9) ; l'appareil de traitement sanguin extracorporel (1) comprenant un pont de raccordement (16, 16') s'étendant entre le conduit d'effluent (12), et/ou le conduit de dialysat facultatif (11), et le conduit de dérivation (5), le pont de raccordement (16, 16') ayant une première extrémité de jonction (16a) positionnée au niveau du conduit d'effluent (12), et/ou au niveau du conduit de dialysat facultatif (11), et une seconde extrémité de jonction (16b) positionnée au niveau du conduit de dérivation (5) ; le pont de raccordement (16, 16') étant pourvu d'une vanne d'écoulement (17) ; l'unité de commande (19) étant reliée à la vanne d'écoulement (17) du pont de raccordement (16, 16') pour piloter cette dernière entre un état ouvert, le pont de raccordement (16, 16') étant en communication fluidique avec le conduit de dérivation (5), et un état fermé, le pont de raccordement (16, 16') n'étant pas en communication fluidique avec le conduit de dérivation (5), facultativement le pont de raccordement (16, 16') étant pourvu d'un filtre (20).

6. Appareil (1) selon la revendication 5, l'unité de commande (19) étant configurée pour maintenir la vanne d'écoulement (17) dans l'état ouvert et le régulateur de débit de fluide (7) dans la première condition de fonctionnement (i) ou dans la seconde condition de fonctionnement (ii), de sorte qu'un fluide provenant du conduit d'effluent (12), et/ou au niveau du conduit de dialysat facultatif (11), s'écoule à travers le pont de raccordement (16, 16') dans le conduit de dérivation (5) vers le capteur (6) et vers le conduit de retour de sang (3b) ou vers le conduit de prélèvement (3a), le capteur (6) détectant l'au moins une substance (S) dans le fluide provenant du conduit d'effluent (12) et/ou au niveau du conduit de dialysat facultatif (11) et s'écoulant à travers le conduit de dérivation (5).

7. Appareil (1) selon la revendication 5 ou 6, le conduit de dérivation (5) étant pourvu d'au moins une vanne de transit (18) interposée entre la première extrémité de jonction (5a) ou la seconde extrémité de jonction (5b) du conduit de dérivation (5) et la seconde extrémité de jonction (16b) du pont de raccordement (16, 16'), l'unité de commande (19) étant reliée à la vanne de transit (18) du conduit de dérivation (5) pour piloter cette dernière entre un état ouvert, le conduit de dérivation (5) étant en communication fluidique avec le conduit de prélèvement de sang (3a) ou avec le conduit de retour de sang (3b), et un état fermé, le conduit de dérivation (5) n'étant pas en communication fluidique avec le conduit de prélèvement de sang (3a) ou avec le conduit de retour de sang (3b).

8. Appareil (1) selon l'une quelconque des revendications 1 à 7, le capteur (6) étant situé entre le régulateur de débit de fluide (7) et la première extrémité de jonction (5a) ou entre le régulateur de débit de fluide (7) et la seconde extrémité de jonction (5b).

9. Appareil (1) selon la revendication 8 lorsqu'elle dépend de la revendication 5, 6 ou 7, le capteur (6) étant situé entre le régulateur de débit de fluide (7) et la seconde extrémité de jonction (16b) du pont de raccordement (16, 16') ou le régulateur de débit de fluide (7) étant situé entre le capteur (6) et la seconde extrémité de jonction (16b) du pont de raccordement (16, 16').

10. Appareil (1) selon la revendication 2, 3 ou 4 ou selon l'une quelconque des revendications 5 à 9 lorsqu'elles dépendent de la revendication 2, 3 ou 4, le conduit de perfusion (13, 21) comprenant : un conduit de pré-perfusion (13) relié au conduit de prélèvement de sang (3a) en un point de jonction (13a) ;
la source de perfusion (14, 22) comprenant : une source de fluide de pré-perfusion (14) d'une solution de pré-perfusion (I) reliée au conduit de pré-perfusion (13) ; la pompe de perfusion (15, 23) comprenant : une pompe de pré-perfusion (15) active sur le conduit de pré-perfusion (13), l'unité de commande (19) étant configurée pour commander la pompe de pré-perfusion (15) entre la condition activée, la pompe de pré-perfusion (15) amenant la solution de pré-perfusion (I) à s'écouler de la source de fluide de pré-perfusion correspondante (14) vers le conduit de prélèvement de sang (3a) du circuit sanguin extracorporel (3), et la condition inactivée, la pompe de pré-perfusion (15) étant inactive et la solution de pré-perfusion (I) ne s'écoulant pas de la source de fluide de pré-perfusion correspondante (14) à travers le conduit de pré-perfusion correspondant (13), la première extrémité de jonction (5a) du conduit de dérivation (5) se raccordant directement au conduit de pré-perfusion (13) entre le point de jonction (13a) et la pompe de pré-perfusion (15) du conduit de pré-perfusion (13).

11. Appareil (1) selon la revendication 10 lorsqu'elle dépend de la revendication 5, 6 ou 7, la seconde extrémité de jonction (16b) du pont de raccordement (16) étant interposée entre le capteur (6) et la première extrémité de jonction (5a) du conduit de dérivation (5) ; facultativement, la seconde extrémité de jonction (16b) du pont de raccordement (16) étant interposée entre le régulateur de débit de fluide (7) du conduit de dérivation (5) et la première extrémité de jonction (5a) du conduit de dérivation (5) et le capteur (6) étant interposé entre le régulateur de débit de fluide (7) du conduit de dérivation (5) et la seconde extrémité de jonction (5b) du conduit de dérivation (5).

12. Appareil (1) selon la revendication 2, 3, 4, 10 ou 11 ou selon l'une quelconque des revendications 5 à 9 lorsqu'elles dépendent de la revendication 2, 3 ou 4, le conduit de perfusion (13, 21) comprenant : un conduit de post-perfusion (21) relié au conduit de retour de sang (3b) en un point de raccordement (21a) ;
la source de perfusion (14, 22) comprenant : une source de fluide de post-perfusion (22) d'une solution de post-perfusion (II) reliée au conduit de post-perfusion (21) ;
la pompe de perfusion (15, 23) comprenant : une pompe de post-perfusion (23) active sur le conduit de post-perfusion (21), l'unité de commande (19) étant configurée pour commander la pompe de post-perfusion (23) entre la condition activée, la pompe de post-perfusion (23) amenant la solution de post-perfusion (II) à s'écouler de la source de fluide de post-perfusion correspondante (22) vers le conduit de retour de sang (3b) du circuit sanguin extracorporel (3), et la condition inactivée, la pompe de post-perfusion (23) étant inactive et la solution de post-perfusion (II) ne s'écoulant pas de la source de fluide de post-perfusion correspondante (22) à travers le conduit de post-perfusion correspondant (21), la seconde extrémité de jonction (5b) du conduit de dérivation (5) se raccordant directement au conduit de post-perfusion (21) entre le point de raccordement (21a) et la pompe de post-perfusion (23) du conduit de post-perfusion (21).

13. Appareil (1) selon la revendication 12 lorsqu'elle dépend de la revendication 10 ou 11 :
dans la première condition de fonctionnement (i), l'unité de commande (19) étant configurée pour maintenir la pompe de pré-perfusion (15) dans la condition inactivée, la pompe de post-perfusion (23) dans la condition activée ou dans la condition inactivée et à commander le régulateur de débit de fluide (7) afin de faire circuler le sang dans le conduit de prélèvement de sang (3a) du circuit sanguin extracorporel (3) à travers le conduit de pré-perfusion (13), le long du conduit de dérivation (5) vers le capteur (6) et vers le conduit de retour de sang (3b) par l'intermédiaire du conduit de post-perfusion (21), le capteur (6) surveillant la concentration de l'au moins une substance (S) dans le sang provenant du conduit de prélèvement de sang (3a) ;
dans la seconde condition de fonctionnement (ii), l'unité de commande (19) étant configurée pour maintenir la pompe de pré-perfusion (15) dans la condition inactivée ou dans la condition activée, la pompe de post-perfusion (23) dans la condition inactivée et à commander le régulateur de débit de fluide (7) afin de faire circuler le sang dans le conduit de retour de sang (3b) du circuit sanguin extracorporel (3) à travers le conduit de post-perfusion (21), le long du conduit de dérivation (5) vers le capteur (6) et vers le conduit de prélèvement de sang (3a) par l'intermédiaire du conduit de pré-perfusion (13), le capteur (6) surveillant la concentration de l'au moins une substance (S) dans le sang provenant du conduit de retour de sang (3b).

14. Appareil (1) selon l'une quelconque des revendications 1 à 13, le régulateur de débit de fluide (7) comprenant une pompe de dérivation (7a) active sur le conduit de dérivation (5) pour générer au moins un écoulement de fluide dans le conduit de dérivation (5) au moins selon la première condition de fonctionnement (i) et la seconde condition de fonctionnement (ii).

15. Appareil (1) selon la revendication 14, la pompe de dérivation (7a) étant une pompe occlusive et/ou au moins une vanne de dérivation étant couplée à la pompe de dérivation (7a), agissant sur le conduit de dérivation (5) et étant commandée par l'unité de commande (19).
